# EUROPEAN PATENT APPLICATION

(11) **EP 4 491 717 A1**
(43) Date of publication of application: **15.01.2025**
(21) Application number: 23766874.4
(22) Date of filing: 08.03.2023
(51) Int. Cl.: C12N 5/10, C12N 7/01, C12N 15/35, C12N 15/864

(54) **PRODUCER CELL, METHOD FOR PRODUCING PRODUCER CELL, AND METHOD FOR PRODUCING ADENO-ASSOCIATED VIRUS**

(30) Priority: 08.03.2022 JP 2022035100; 20.02.2023 JP 2023024167
(71) Applicant: FUJIFILM Corporation, Tokyo 106-8620 (JP)
(72) Inventor: MURAGUCHI, Taichi, Ashigarakami-gun, Kanagawa 258-8577 (JP); MORI, Yusuke, Ashigarakami-gun, Kanagawa 258-8577 (JP); UMETANI, Sayako, Ashigarakami-gun, Kanagawa 258-8577 (JP)
(74) Representative: Hoffmann Eitle
(86) International application number: PCT/JP2023/008736
(87) International publication number: WO 2023/171698

(57) **Abstract**

An object of the present invention is to provide a producer cell for AAV production, in which a cell injury at the time of establishing a cell strain is avoided or suppressed, a manufacturing method of the producer cell, and a manufacturing method of an adeno-associated virus using the producer cell. According to the present invention, there is provided a producer cell containing, in a chromosome, a Cap gene under a control of an exogenous promoter and a Rep gene under a control of an exogenous promoter, in which the producer cell does not contain at least one of a VA-RNA gene or an E4 gene, and the producer cell is a mammalian cell.

## Description

### Technical Field

The present invention relates to a producer cell for manufacturing an adeno-associated virus. The present invention further relates to a manufacturing method of the producer cell and a manufacturing method of an adeno-associated virus using the producer cell.

### Background Art

The adeno-associated virus (also referred to as AAV) is a linear single-stranded DNA virus belonging to Parvoviridae. The wild-type AAV genome contains a replication regulatory gene (Rep gene) and a capsid structural gene (Cap gene), and inverted terminal repeat sequences (ITR) are adjacent to these genes for viral replication and packaging. The AAV vector can transfer a gene to proliferating and non-proliferating cells, and can be expressed for a long term particularly in non-dividing cells. In addition, AAV is considered to be non-pathogenic and has low immunogenicity. From the above, the AAV vector has been clinically applied as a vector for gene therapy.

AAV is a non-enveloped virus that proliferates in the presence of helper viruses such as adenovirus and herpesvirus. In the preparation of AAV to be used for gene therapy or nucleic acid transfer, classically, AAV replication has been performed by co-infecting host cells with an adenovirus. In addition, a gene responsible for the helper action of adenovirus has been clarified, and a plasmid bearing this gene has also been used. For example, a plasmid containing a Rep gene and a Cap gene, an adenovirus helper plasmid, and a plasmid containing a target gene (a transgene) are simultaneously transfected into HEK293 cells, and can thus be packaged as recombinant AAV (rAAV).

Patent Document 1 describes that the AAV is produced using mammalian cells containing a nucleic acid molecule encoding a virus helper gene under the control of a first derepressible promoter, a nucleic acid molecule encoding an AAV gene under the control of a second derepressible promoter, and a nucleic acid molecule encoding a repressor element of a first and second derepressible promoter.

Patent Document 2 describes a host cell containing a nucleic acid encoding an adeno-associated virus REP protein REP78 and REP68, in which an internal AAV promoter p19 is inactivated by one or more mutations maintaining the functionality of the REP78 protein and the REP68 protein.

### Prior Art Documents

### Patent Documents

Patent Document 1: WO2020-132059A
Patent Document 2: WO2019-57691A

### SUMMARY OF THE INVENTION

### Object to be solved by the invention

In the method of Patent Document 1, there is a problem that, since the control sequence is added to each of the helper gene and the AAV gene required for AAV production, it may be difficult to sufficiently control the expression level for each target gene because of mutual interference between three to five control sequences.

In addition, as an example of a method for producing AAV, there is a method using a producer cell. In a case of establishing a producer cell that produces AAV, there is a problem that a cell injury occurs. In Patent Document 2, as a method for avoiding the cytotoxicity of the REP protein, a method of inactivating the internal AAV promoter p19 is described, but the manufacture of the producer cell is not described.

An object of the present invention is to provide a producer cell for AAV production, in which a cell injury at the time of establishing a cell strain is avoided or suppressed. Another object of the present invention is to provide a manufacturing method of the producer cell and a manufacturing method of an adeno-associated virus using the producer cell.

### Means for solving the object

As a result of intensive studies to solve the above-described object, the present inventors have found that, by establishing the producer cell using the Cap gene under the control of an exogenous promoter and the Rep gene under the control of an exogenous promoter and without using at least one of the VA-RNA gene or the E4 gene, it is possible to avoid or reduce the cell injury at the time of establishing the producer cell. The present invention has been completed based on the above findings.

According to an aspect of the present invention, the following inventions are provided.
<1> A producer cell comprising, in a chromosome: a Cap gene under a control of an exogenous promoter; and a Rep gene under a control of an exogenous promoter, in which the producer cell does not contain at least one of a VA-RNA gene or an E4 gene, and the producer cell is a mammalian cell.
<2> The producer cell according to <1>, in which the exogenous promoter of the Rep gene and the exogenous promoter of the Cap gene are the same promoter.
<3> The producer cell according to <1>, in which the Rep gene is present on a downstream side of the Cap gene in a transcription direction.
<4> The producer cell according to <1>, in which the producer cell contains the E4 gene under a control of an exogenous promoter in the chromosome, and the producer cell does not contain the VA-RNA gene.
<5> The producer cell according to <4>, in which the E4 gene is present on a downstream side of an IRES.
<6> The producer cell according to <4>, in which the exogenous promoter of the E4 gene is an inducible promoter capable of switching on and off.
<7> The producer cell according to <6>, in which the exogenous promoter of the E4 gene is a promoter capable of inducing expression by a drug.
<8> The producer cell according to <7>, in which the exogenous promoter of the E4 gene is present on a downstream side of a tetracycline response element.
<9> The producer cell according to <1>, in which the producer cell does not contain both the VA-RNA gene and the E4 gene.
<10> The producer cell according to any one of <1> to <9>, further comprising an E2A gene.
<11> The producer cell according to <10>, in which the E2A gene is present on a downstream side of an IRES.
<12> The producer cell according to <10>, in which the E2A gene is under a control of an exogenous promoter.
<13> The producer cell according to <12>, in which the E2A gene is under a control of a cytomegalovirus-derived promoter.
<14> The producer cell according to <10>, in which an exogenous promoter of the E2A gene is an inducible promoter capable of switching on and off.
<15> The producer cell according to <14>, in which the exogenous promoter of the E2A gene is a promoter capable of inducing expression by a drug.
<16> The producer cell according to <15>, in which the exogenous promoter of the E2A gene is present on a downstream side of a tetracycline response element.
<17> A producer cell comprising, in a chromosome, a Cap gene under a control of an exogenous promoter, a Rep gene under a control of an exogenous promoter, and an E4 gene under a control of an exogenous promoter, in which the producer cell does not contain a VA-RNA gene, and the producer cell is a mammalian cell.
<18> The producer cell according to any one of <1> to <17>, further comprising a target gene.
<19> The producer cell according to any one of <1> to <18>, in which one or more of the exogenous promoter of the Cap gene and the exogenous promoter of the Rep gene are an inducible promoter capable of switching on and off.
<20> The producer cell according to <19>, in which the one or more of the exogenous promoter of the Cap gene and the exogenous promoter of the Rep gene are a promoter capable of inducing expression by a drug.
<21> The producer cell according to <20>, in which the one or more of the exogenous promoter of the Cap gene and the exogenous promoter of the Rep gene is present on a downstream side of a tetracycline response element.
<22> The producer cell according to any one of <1> to <21>, further comprising a Small Rep gene under a control of an exogenous promoter.
<23> The producer cell according to <22>, in which a Rep gene is present on a downstream side of the Small Rep gene in a transcription direction.
<24> The producer cell according to <22>, in which the exogenous promoter of the Small Rep gene is an inducible promoter capable of switching on and off.
<25> The producer cell according to <24>, in which the exogenous promoter of the Small Rep gene is a promoter capable of inducing expression by a drug.
<26> The producer cell according to <25>, in which the exogenous promoter of the Small Rep gene is present on a downstream side of a tetracycline response element.
<27> The producer cell according to any one of <1> to <26>, further comprising a gene that expresses a reverse tetracycline-controlled transactivator.
<28> The producer cell according to <27>, in which the gene that expresses the reverse tetracycline-controlled transactivator is present on a downstream side of the Rep gene.
<29> The producer cell according to <1>, in which the producer cell contains an E4 gene under a control of an exogenous promoter, an E2A gene under a control of an exogenous promoter, and a Small Rep gene under a control of an exogenous promoter, and the exogenous promoter of the Cap gene and an exogenous promoter of the E2A gene are the same promoter, and the exogenous promoter of the Rep gene and the exogenous promoter of the Small Rep gene are the same promoter.
<30> A producer cell comprising, in a chromosome, a Cap gene under a control of an exogenous promoter, a Rep gene under a control of an exogenous promoter, an E4 gene under a control of an exogenous promoter, and an E2A gene under a control of an exogenous promoter, in which the producer cell does not contain a VA-RNA gene, the producer cell is a mammalian cell, the Cap gene, the Rep gene, and an E2A gene are present in a vicinity of each other, and the E4 gene is not present in the vicinity of the Cap gene, the Rep gene, and the E2A gene.
<31> A producer cell comprising, in a chromosome, a first vector carrying a Cap gene under a control of an exogenous promoter, a Rep gene under a control of an exogenous promoter, and an E2A gene under a control of an exogenous promoter, and a second vector carrying an E4 gene under a control of an exogenous promoter, in which the producer cell does not contain a VA-RNA gene, and the producer cell is a mammalian cell.
<32> The producer cell according to <31>, in which the number of E4 gene insertions is smaller than the number of insertions of other genes, on the chromosome.
<33> The producer cell according to <31> or <32>, in which the number of E4 gene insertions on the chromosome is smaller than the number of Cap gene insertions on the chromosome or the number of E2 gene insertions on the chromosome.
<34> The producer cell according to <31> or <32>, in which the number of E4 gene insertions on the chromosome is 0.4 times or less the number of Cap gene insertions on the chromosome.
<35> The producer cell according to <31> or <32>, in which the number of E4 gene insertions on the chromosome is 0.4 times or less the number of E2 gene insertions on the chromosome.
<36> A manufacturing method of a producer cell, which is a manufacturing method of the producer cell according to any one of <1> to <35>, the manufacturing method comprising transferring the Cap gene under the control of the exogenous promoter and the Rep gene under the control of the exogenous promoter into a mammalian cell.
<37> The manufacturing method of a producer cell according to <36>, further comprising introducing a reverse tetracycline-controlled transactivator.
<38> A manufacturing method of an adeno-associated virus, comprising culturing the producer cell according to any one of <1> to <35>, to produce an adeno-associated virus.
<39> A manufacturing method of an adeno-associated virus, the method comprising a step of culturing the producer cell according to any one of <1> to <35> under a condition in which expression of a Cap gene and expression of a Rep gene are not caused to be induced to proliferate the producer cell, and a step of culturing the proliferated producer cell under a condition in which expression of a Cap gene and expression of a Rep gene are induced, to produce an adeno-associated virus.
<40> A manufacturing method of an adeno-associated virus, comprising a step of culturing the producer cell according to any one of <4> to <8> under a condition in which expression of the Cap gene, expression of the Rep gene, and expression of the E4 gene are not caused to be induced, to proliferate the producer cell and a step of culturing the proliferated producer cell under a condition in which the expression of the Cap gene, the expression of the Rep gene, and the expression of the E4 gene are induced, to produce an adeno-associated virus.
<A> An adeno-associated virus manufactured by the manufacturing method of an adeno-associated virus according to any one of <38> to <40>.
<B> A kit of vectors for manufacturing a producer cell, the kit comprising a first vector carrying a Cap gene under a control of an exogenous promoter, a Rep gene under a control of an exogenous promoter, and an E2A gene under a control of an exogenous promoter and a second vector carrying an E4 gene under the control of an exogenous promoter.

### Effect of the invention

According to the present invention, it is possible to avoid or reduce the cell injury at the time of establishing the producer cell.

### BRIEF DESCRIPTION OF THE DRAWINGS

Fig. 1 shows the results of transferring a Rep gene and a Cap gene into a cell and evaluating expression of REP and CAP.
Fig. 2 shows the results of transferring the Rep gene and the Cap gene under the control of an exogenous promoter into a cell and evaluating the AAV production ability.
Fig. 3 shows the results of evaluating the expression of REP and CAP after the Rep gene and the Cap gene were transferred into a cell and the expression was then induced by adding doxycycline.
Fig. 4 shows the results of Western blot using an anti-E2 antibody and GFP observation for cells into which an E2 gene and a green fluorescent protein (GFP) gene have been transferred.
Fig. 5 shows the results of transferring the Rep gene and the Cap gene into a cell having the E2 gene and the GFP gene and evaluating the AAV production ability of the obtained cell.
Fig. 6 shows an outline of Experiment 6.
Fig. 7 shows a quantification result of AAV for a producer cell strain selected using a neomycin resistant gene as a selection marker.
Fig. 8 shows the quantification results of AAV for a producer cell strain selected using red fluorescent protein (RFP) as a selection marker.
Fig. 9 shows microscopic views of a producer cell strain.

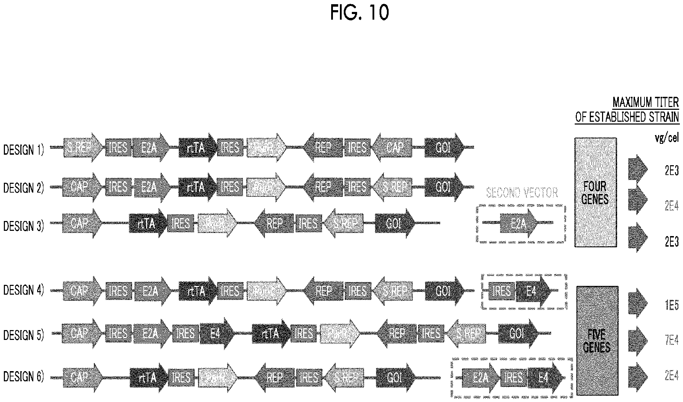

Fig. 10 is a schematic diagram of a gene design of Experiment 7. GOI represents a GFP gene.
Fig. 11 shows an outline of Experiment 7 and Experiment 8.
Fig. 12 shows the quantification results of AAV for the highest titer strain of each of the designs among HEK293 containing each arrangement of Designs 1 to 6 in the chromosome.
Fig. 13 shows the quantification results of the number of genes of each of Rep, Cap, E2, and E4 on the chromosome for a plurality of clones of HEK293 established using the vector set of Design 4 of Experiment 7.
Fig. 14 shows the quantification results of the number of genes of each of Rep, Cap, E2, and E4 on the chromosome for a plurality of clones of HEK293 established using the vector of Design 5 of Experiment 7.
Fig. 15 shows the quantification results of AAV for the HEK293 cell strain containing the arrangement of Design 4 in a chromosome and the HEK293 cell strain containing the arrangement of Design 5 in the chromosome, which have been acclimatized in a suspension culture.

### DESCRIPTION OF THE PREFERRED EMBODIMENTS

Hereinafter, an example of the embodiment of the present disclosure will be described. However, the present disclosure is not limited to the following embodiments, and can be implemented with appropriate changes within the scope of the object of the present disclosure. In the present specification, the numerical range indicated by using "to" means a range including numerical values described before and after "to" as a minimum value and a maximum value, respectively.

### <Explanation of terms>

A promoter indicates a sequence that drives gene expression and means a DNA regulatory region/sequence to which an RNA polymerase can be bound and which is involved in the initiation of transcription of a downstream coding sequence or a downstream non-coding sequence.

The exogenous promoter means a promoter not contained in the wild-type AAV gene. Specifically, the exogenous promoter is a promoter not known to allow the AAV gene to be expressed under natural conditions.

The selection marker refers to a gene for selecting a cell that expresses a nucleic acid sequence vigorously. Examples of a suitable selection marker include a gene encoding resistance to antibiotics such as kanamycin, neomycin, puromycin, hygromycin, blasticidin, or zeocin. Another example of the suitable selection marker is a fluorescent protein, for example, a green fluorescent protein (GFP), a red fluorescent protein (RFP), or a blue fluorescent protein (BFP).

The vector is a DNA or RNA molecule that is used to artificially transport an exogenous gene to another cell. A vector for expressing a gene is referred to as an expression vector. In a case where a vector containing an exogenous gene to be transferred is transferred into a cell, the exogenous gene is replicated and/or expressed in the cell. Examples of the vector include an episomal (for example, plasmid) vector and a non-episomal vector. The vector can be transferred into a host cell by methods such as transfection, transduction, cell fusion, lipofection, and electroporation.

The term "bearing" means that another nucleic acid sequence is incorporated into a chromosome or a nucleic acid sequence.

The gene is not limited as long as it is a base sequence, but a gene having a function in a living body is preferable. The gene may be, for example, a nucleotide encoding a polypeptide, and examples thereof include a nucleic acid molecule, such as cDNA or a genomic DNA.

The producer cell is a cell that can produce rAAV without requiring additional helper functions such as the transfer of a helper gene or virus infection. In the present specification, the producer cell is a cell in which a Rep gene and a Cap gene have been incorporated into a chromosome. Furthermore, additionally, the target gene (for example, a gene for desired treatment or prevention) and the necessary adenovirus helper gene may be incorporated into the chromosome. Target gene may contain an ITR. In addition, the producer cell may be a cell into which a gene containing the target gene has been incorporated adjacent to two AAV inverted terminal repeats (ITR). A gene not incorporated into a chromosome may be transferred to a producer cell by being carried by a vector to produce rAAV.

In addition, the producer cell may be a cell in which a gene expressing a factor that acts on the activity of the promoter is incorporated into a chromosome, or may be a cell in which all the genes of the factors that act on the activity of the promoter are incorporated into a chromosome. The factor that acts on the activity of the promoter is preferably a factor that activates the promoter, and more preferably a reverse tetracycline-controlled factor.

### <Producer cell>

The producer cell according to the embodiment of the present invention contains, in a chromosome, a Cap gene under a control of an exogenous promoter and a Rep gene under a control of an exogenous promoter, in which the producer cell does not contain at least one of a VA-RNA gene or an E4 gene, and the producer cell is a mammalian cell.

In Experiment 1 of Examples of the present invention, it was confirmed that the expression levels of REP and CAP were significantly reduced under the condition in which the E4 gene or the VA-RNA gene is not used.

In Experiment 2, the mandatory (constitutive) CMV promoter is directly bonded to the Rep gene and the Cap gene to be expressed. As a result, the production of AAV is possible even under the condition in which the E4 gene and the VA-RNA gene are not used. However, since REP has cytotoxicity, the Rep gene cannot be incorporated as it is to obtain a stable strain.

In Experiment 3, a drug-inducible type (TRE-CMV-minimal promoter) is linked to the Rep gene and the Cap gene. As a result, even under the condition in which the E4 gene and the VA-RNA gene are not used, REP and CAP could be expressed.

In Experiment 4, a cell strain stably having the E2 gene and the GFP gene (target gene) is established.

In Experiment 5, the Rep gene and the Cap gene are transferred into the cell strain established in Experiment 4. It is confirmed that the obtained cell strain can produce AAV.

In Experiment 6, the switched-on Rep gene and Cap gene are incorporated into the cells. A cell strain stably having the Rep gene and the Cap gene is established by selecting cells using a drug or fluorescence as an indicator. The obtained cell strain is confirmed to be able to produce AAV.

In Experiment 7, the Rep gene, the Cap gene, the E2 gene, and in some cases, the E4 gene are incorporated into the cells. A cell strain stably having the Rep gene and the Cap gene is established by selecting cells using a drug or fluorescence as an indicator. The obtained cell strain is confirmed to be able to produce AAV.

In Experiment 8, the producer cells established in Experiment 7 are floated and cultured. It is confirmed that the floated cells can produce AAV.

The producer cell according to the embodiment of the present invention has a Cap gene under the control of an exogenous promoter and a Rep gene under the control of an exogenous promoter in a chromosome.

As the Rep gene and the Cap gene, a Rep gene and a Cap gene of an adeno-associated virus gene can be used.

Adeno-associated virus (AAV) refers to a small, incompletely replicative, non-enveloped virus containing single-stranded DNA from the family of Parvoviridae and Dependoparvovirus. It is known that there are more than 100 serum types in AAV, and that the host range and the characteristics of the virus differ depending on the difference in the serum types. Serum type 2 (AAV2) is one of the serum types that have been widely studied for a long time, and it is known that the host range is very wide. The serum type 1 (AAV1), the serum type 5 (AAV5), and the serum type 6 (AAV6) are serum types having higher tissue directivity. It is said that AAV1 has high gene transfer efficiency into muscle, liver, airway, central nervous system, and the like, AAV5 has high gene transfer efficiency into central nervous system, liver, retina, and the like, and AAV6 has high gene transfer efficiency into heart, muscle, liver, and the like. In the present invention, the serum type 2 or the serum type 5 is preferably used. The serum type 5 is particularly preferable.

The adeno-associated virus gene refers to a gene composed of one or a plurality of nucleic acid sequences derived from the serum types of one or a plurality of adeno-associated viruses. The adeno-associated virus gene is preferably a gene involved in replication and packaging of AAV and a gene encoding an AAV constituent protein.

The Rep gene and the Cap gene encode proteins involved in the replication and packaging of the virion. The Cap region expresses VP1, VP2, and VP3.

The Rep gene and the Cap gene may be a wild-type gene, but a gene in which modifications such as substitution, deletion, insertion, or addition of a base have been made to the wild-type gene may be used as long as it exhibits an inherent function.

In a case where the wild-type Rep gene and wild-type Cap gene are modified by substitution, deletion, insertion, addition, or the like of a base, the number of the modified bases is preferably 1 to 20, more preferably 1 to 10, and still more preferably 1 to 3. The base sequences of the modified Rep gene and the modified Cap gene preferably exhibit 85% or more of the sequence identity, more preferably exhibit 90% or more of the sequence identity, still more preferably exhibit 95% or more of the sequence identity, and even still more preferably exhibit 98% or more of the sequence identity, with the base sequences of the wild-type Rep gene and the wild-type Cap gene.

In a case where the Rep gene and the Cap gene are transferred into a cell, a vector containing the Rep gene and the Cap gene can be transferred into the cell. The arrangement of the Rep gene and the Cap gene in the vector is not particularly limited, and the Cap gene and the Rep gene may be arranged in the same direction or may be arranged in different directions to each other. Preferably, the Cap gene and the Rep gene are arranged in different orientations to each other in the vector. The orientation of a certain gene means a direction from the 5' side to the 3' side, and the arrangement in the different orientation means that the direction from the 5' side to the 3' side of a certain gene and the other gene is arranged in the opposite direction on the vector. In addition, the Rep gene and the Cap gene may be arranged in different vectors.

The Rep gene means a region of the AAV genome encoding a virus replication protein that is known to those skilled in the art and that is collectively required for replication of a virus genome, or its functional homolog, for example, a human herpesvirus 6 (HHV-6) Rep gene, or the like (mediating AAV-2 DNA replication is known). Therefore, the coding region of the Rep gene includes at least a gene encoding REP78 and REP68 (long form of REP protein) and REP52 and REP40 (short form of REP protein; also referred to as "Small Rep") of AAV or a functionally homologous thereof. The Small Rep gene includes, for example, at least a gene encoding REP52 or a gene encoding REP40, and preferably includes both. For the Rep gene and the SmallRep gene, Maurer AC, Weitzman MD. Adeno-Associated Virus Genome Interactions Important for Vector Production and Transduction. Hum Gene Ther. 2020 May; 31(9-10): 499-511. doi: 10.1089/hum.2020.069. PMID: 32303138; PMCID: PMC7232694; and Keiya Ozawa. Gene therapy using AAV. Virus Vol. 57, No. 1, pp. 47-56, 2007 are referred to and incorporated in the present specification.

The production of AAV requires REP68 or REP78, and REP68 and REP78 are translation products of two types of mRNAs transcribed from the same gene by alternative splicing. In the present invention, the REP includes at least REP68 or REP78 protein, and may preferably further include REP52 and/or REP40. The coding region of the Rep gene used in the present invention may be derived from any AAV serum type, but is preferably derived from AAV2. Examples of those derived from AAV2 include REP78 and REP68, and REP52 and REP40, and ITR.

The Cap gene means a region in the AAV genome encoding a capsid protein of a virus known to those skilled in the art. Examples of these capsid proteins are AAV capsid proteins VP1, VP2 and VP3. The Cap gene used in the present invention may be derived from any AAV serum type, but is preferably derived from AAV2 or AAV5. AAV5 is particularly preferable.

Preferably, the exogenous promoter of the Cap gene is an inducible promoter capable of switching on and off.

Preferably, the exogenous promoter of the Rep gene is an inducible promoter capable of switching on and off.

As the inducible promoter capable of switching on and off, a promoter capable of regulating the on and off of expression depending on the presence or absence of a stimulus can be used. Examples of the stimulus include chemical stimuli (intrinsic hormone/stress response, lactose, tetracycline or a derivative thereof (for example, doxycycline), cumic acid, a protein (rapamycin, FKCsA, abscisic acid (ABA), and the like), tamoxifen/Cre-loxP (a system using a Cre promoter modified such that activation can be induced by tamoxifen), and a riboswitch), and a physical stimulus (blue light, heat), but the stimulus is not particularly limited.

The inducible promoter capable of switching on and off is preferably a promoter capable of inducing expression by a drug. The drug is preferably tetracycline or a derivative thereof (for example, doxycycline).

Examples of the inducible promoter capable of switching on and off include a tetracycline-responsive promoter, a RU486-inductive promoter, an ecdysone-inductive promoter, a rapamycin-inductive promoter, and a metallothionein promoter. Specific examples of the tetracycline-responsive promoters include a TRE-CMV-minimal promoter consisting of a tetracycline response element and a part of a CMV promoter. This sequence is a promoter in which a tetracycline response element and a part of the CMV promoter are linked. This promoter is used in a Tet on/off system (manufactured by TET systems).

The insulator is a sequence for stably controlling the transcription of a gene without being affected by the surrounding sequence. It is preferable that the insulator is present between the REP and the CAP. In addition, the insulator is not particularly limited, but is preferably 50 bp or more and 10,000 bp or less, more preferably 100 bp or more and 5,000 bp or less, and particularly preferably 300 bp or more and 4,000 bp or less. bp (base pair) means a base sequence or the number of base sequences.

An internal ribosome entry site (IRES) is a sequence that causes to initiate translation of a transcription product of a gene, and by linking a certain gene and another gene with an IRES, each gene can be translated from the same transcription product. The IRES can be encoded between the Cap gene and the E2 gene.

The inducible promoter capable of switching on and off may be a Tet-on/off system which is a promoter capable of regulating expression by tetracycline, or a Tet-on system.

In the Tet-on system, the promoter additionally contains at least one tetracycline response element (Tet operon). By using a tetracycline response element (Tet operon: tetracycline-controlled transcriptional activation), transcription can be reversibly switched on or off in the presence of one of the tetracycline which is an antibiotic, or a derivative thereof (for example, doxycycline). The Tet repressor protein present in the cell blocks expression by binding to a Tet operator sequence introduced into a promoter. Therefore, no gene expression is observed in a case where the Tet repressor binds to the Tet operator sequence. By adding tetracycline or doxycycline, the Tet repressor is sequestered to allow promoter activity and turn on gene expression. The Tet operon system is widely available, such as the Tet operon used in the pcDNA (trademark) 4/TO mammalian expression vector available from Thermo Fisher Scientific Inc.

The promoter is preferably present on a downstream side of the tetracycline response element.

Preferably, the producer cell according to the embodiment of the present invention may include a factor that acts on the activity of the promoter, and includes a gene that expresses preferably a factor that activates the promoter and more preferably a reverse tetracycline-controlled transactivator (rtTA). Preferably, the gene expressing the reverse tetracycline-controlled transactivator is present on a downstream side of the Rep gene.

The Rep gene and the Cap gene may be driven by one and the same exogenous promoter, or the Rep gene and the Cap gene may be driven by two exogenous promoters driving each of the genes.

In a case where the Rep gene and the Cap gene may be driven by two exogenous promoters driving each of the genes, the exogenous promoter of the Rep gene and the exogenous promoter of the Cap gene may be promoters having the same sequence or may be promoters having different sequences, but are preferably promoters having the same sequence.

In a case where the Cap gene and the Rep gene are contained in the same vector, the Rep gene may be present on a downstream side of the Cap gene in the transcription direction, or the Rep gene may be present on an upstream side of the Cap gene in the transcription direction, but the Rep gene is preferably present on a downstream side of the Cap gene in the transcription direction.

The producer cell according to the embodiment of the present invention may preferably further contain a Small Rep gene under the control of an exogenous promoter. Examples of the Small Rep gene include a gene encoding REP52 and a gene encoding REP40.

In a case where the producer cell according to the embodiment of the present invention contains the Small Rep gene under the control of the exogenous promoter, the Small Rep gene is preferably present on an upstream side of the Rep gene.

The Small Rep gene is preferably an inducible promoter capable of switching on and off. Details of the inducible promoter capable of switching on and off are as described above in the present specification.

The exogenous promoter of the Small Rep gene is preferably a promoter capable of inducing expression by a drug. The details of the promoter capable of inducing expression by a drug are as described above in the present specification.

The exogenous promoter of the Small Rep gene is preferably present on a downstream side of the tetracycline response element.

The vector containing the Rep gene and the Cap gene is not particularly limited as long as it is a nucleic acid, and may be cyclic or linear. For example, an artificially designed nucleic acid or the like can be used, and the plasmid is preferable.

In the manufacture of the adeno-associated virus, a virus helper gene is generally used. The virus helper gene is a non-adeno-associated virus gene for enabling replication and packaging of an adeno-associated virus. As the virus helper gene, a gene derived from a virus of another species other than the adeno-associated virus is used. Specific examples of the virus helper gene include a virus helper gene derived from an adenovirus or a herpesvirus, and the virus helper gene is preferably derived from an adenovirus.

The adenovirus refers to a non-enveloped virus having an icosahedron nucleocapsid containing double-stranded DNA in a family of Adenoviridae. Over 50 subtypes of adenovirus have been isolated from humans and many additional subtypes have been isolated from other mammals and birds. These subtypes belong to the family of Adenoviridae and are classified into two genera (that is, Mastadenovirus and Aviadenovirus). These adenoviruses are morphologically and structurally similar. However, in humans, adenoviruses exhibit different immunological properties, and that is, are classified into serum types. Two human serum types of adenovirus (that is, Ad2 and Ad5) have been intensively studied.

Examples of the virus helper gene derived from adenovirus include E1A, E1B, E2 (preferably E2A), E4, VA-RNA, and the like. In the host cell having all or a part of the E1 region, the region of the adenovirus genome required for replicating the AAV genome and packaging of the AAV genome into the capsid to form a virus virion is regarded as the E2A region, the E4 region, and the VA1 RNA region. The E2 gene contains an E2A region and an E2B region, but in the E2 gene according to the present invention, only the E2A region can be used. In addition, the E4 gene contains a plurality of ORFs. For the function by the E4 region, the 34 kDa E4 protein encoded by the open reading frame 6 (E4ORF6) of the E4 region is required for replicating the AAV. Therefore, in the E4 gene according to the present invention, only ORF6 can be used.

The producer cell according to the embodiment of the present invention does not contain at least one of a VA-RNA gene or an E4 gene. That is, examples of the producer cell according to the embodiment of the present invention include a case where the producer cell contains the E4 gene and does not contain the VA-RNA gene, a case where the producer cell contains the VA-RNA gene and does not contain the E4 gene, and a case where the producer cell does not contain both the VA-RNA gene and the E4 gene.

Preferably, the producer cell according to the embodiment of the present invention contains an E4 gene under the control of an exogenous promoter in a chromosome and does not contain a VA-RNA gene. The producer cell in this case is a producer cell containing, in a chromosome, a Cap gene under a control of an exogenous promoter, a Rep gene under a control of an exogenous promoter, and an E4 gene under a control of an exogenous promoter, in which the producer cell does not contain a VA-RNA gene, and the producer cell is a mammalian cell.

In a case where the producer cell according to the embodiment of the present invention contains the E4 gene, the E4 gene is preferably present on a downstream side of the IRES.

Preferably, the exogenous promoter of the E4 gene is an inducible promoter capable of switching on and off. Details of the inducible promoter capable of switching on and off are as described above in the present specification.

The exogenous promoter of the E4 gene is preferably a promoter capable of inducing expression by a drug. The details of the promoter capable of inducing expression by a drug are as described above in the present specification.

The exogenous promoter of the E4 gene is preferably present on a downstream side of the tetracycline response element.

In another aspect, the producer cell according to the embodiment of the present invention does not contain both the VA-RNA gene and the E4 gene. The "not containing a certain gene" means not only that the gene is not contained in the chromosome but also that the gene is not contained in the cell.

The producer cell according to the embodiment of the present invention may preferably further contain an E2 gene (preferably, an E2A gene).

The E2A gene may be a wild-type gene, but a gene in which modifications such as substitution, deletion, insertion, or addition of a base have been made to the wild-type gene may be used as long as it exhibits an inherent function.

In a case where the producer cell according to the embodiment of the present invention contains the E2 (preferably E2A) gene, the E2 gene is preferably present on a downstream side of the IRES. Alternatively, the E2 gene may be introduced as a second vector different from the vector containing the Rep gene and the Cap gene.

In a case where modifications such as substitution, deletion, insertion, or addition of a base are made to the wild-type E2A gene, the number of the modified bases is preferably 1 to 20, more preferably 1 to 10, and still more preferably 1 to 3. The base sequence of the modified E2A gene exhibits a sequence identity of preferably 85% or more, more preferably 90% or more, still more preferably 95%, and even still more preferably 98% or more with the base sequence of the wild-type E2A gene.

The E2 (preferably, E2A) gene may be under the control of a promoter, preferably under the control of an exogenous promoter.

The exogenous promoter of the E2 (preferably, E2A) gene is preferably an inducible promoter capable of switching on and off. Details of the inducible promoter capable of switching on and off are as described above in the present specification.

The exogenous promoter of the E2 (preferably, E2A) gene is preferably a promoter capable of inducing expression by a drug. The details of the promoter capable of inducing expression by a drug are as described above in the present specification.

The E2 (preferably, E2A) gene may control on and off of the gene under the control of a tetracycline-responsive promoter (TET). The promoter of the E2 (preferably, E2A) gene is not particularly limited, but examples thereof can include a cytomegalovirus-derived promoter (CMV promoter) (including an enhancer as desired), an SV40 early promoter, a human elongation factor-1α (EF-1α) promoter, a human ubiquitin C promoter, a retrovirus Rous sarcoma virus (RSV) LTR promoter, a dihydrofolate reductase promoter, a β-actin promoter, a phosphoglycerate kinase (PGK) promoter, tetracycline-responsive promoter, and the like. Among the above, a tetracycline-responsive promoter or a cytomegalovirus-derived promoter is preferable. Examples of the tetracycline-responsive promoter include a TRE-CMV-minimal promoter. The exogenous promoter of the E2 (preferably, E2A) gene is preferably present on a downstream side of the tetracycline response element.

In the preferable aspect of the present invention, the producer cell contains the E4 gene under a control of an exogenous promoter, the E2 (preferably, E2A) gene under a control of an exogenous promoter, and the Small Rep gene under a control of an exogenous promoter, and the exogenous promoter of the Cap gene and an exogenous promoter of the E2 gene are the same promoter, and the exogenous promoter of the Rep gene and the exogenous promoter of the Small Rep gene are the same promoter.

In a case of transferring an E2 (preferably, E2A) gene into a cell, a vector containing the E2 (preferably, E2A) gene can be transferred into the cell.

The vector containing the E2 (preferably, E2A) gene is not particularly limited as long as it is a nucleic acid, and may be cyclic or linear. For example, a plasmid, an artificially designed chromosome, or the like can be used, and the plasmid is preferable.

The producer cell according to the embodiment of the present invention may preferably further contain a target gene.

As target gene, for example, a gene for treatment or prevention can be used.

As the gene for treatment or prevention, a gene that is incomplete or lost in the genome of the target cell, a gene encoding non-natural protein having a desired biological or therapeutic effect (for example, antiviral function), or the like can be used, but the gene is not particularly limited. Specific examples of the gene for treatment or prevention include a gene used for treatment or prevention of inflammatory diseases, autoimmunity, chronic and infectious diseases (including disorders such as AIDS, cancer, nervous system diseases, cardiovascular diseases, and hypercholesterolemia), various blood diseases such as anemia and hemophilia, or gene deficiency (for example, cystic fibrosis, Gaucher's disease, adenosine deaminase (ADA) deficiency, emphysema, and the like). In the present example, GFP or RFP is used instead of the target gene.

The gene for treatment or prevention may be several antisense oligonucleotides (for example, a short-chain oligonucleotide complementary to a sequence around a translation initiation site (AUG codon) of mRNA) that are useful in antisense therapy against cancer and viral disease.

The gene for therapy or prophylaxis may be linked to a promoter for expressing the gene for treatment or prevention. The promoter for expressing a gene for treatment or prevention is not particularly limited, but examples thereof can include a cytomegalovirus-derived promoter (including an enhancer as desired), an SV40 early promoter, a human elongation factor-1α (EF-1α) promoter, a human ubiquitin C promoter, a retrovirus Rous sarcoma virus LTR promoter, a dihydrofolate reductase promoter, a β-actin promoter, a phosphoglycerate kinase (PGK) promoter, and the like. The gene for treatment or prevention and the promoter for expressing the gene are preferably flanked by the ITR sequences.

The inverted terminal repeat sequence (ITR) is a base sequence for incorporating the gene of the adeno-associated virus into the target cell. Two inverted terminal repeat sequence are usually present at positions flanking the target gene. The inverted terminal repeat sequence is not limited thereto, but is preferably a serum type 2 AAVderived sequence.

The producer cell according to the embodiment of the present invention is preferably a eukaryotic cell, and is, for example, a mammalian cell or an insect cell. The cell is more preferably a mammalian cell. Examples of the mammalian cell include a human cell, a mouse cell, a rat cell, a monkey cell, and a hamster cell, but the cells are not particularly limited. The human cell can be preferably used. Examples of the cell include a human embryo-derived kidney (HEK293) cell, mouse myeloma (NSO) cell lines, Chinese hamster ovary (CHO) cell lines, HT1080, H9, HepG2, MCF7, MDBK Jurkat, NIH3T3, PC12, a baby hamster kidney (BHK) cell, VERO, SP2/0, YB2/0, Y0, C127, an L cell, COS (for example, COS1 and COS7), QC1-3, VERO, PER. C6, HeLa, EB1, EB2, EB3, and hybridoma cell lines. Preferably, the cell is a HEK 293 cell.

The producer cell according to the embodiment of the present invention contains a Cap gene under the control of an exogenous promoter and a Rep gene under the control of an exogenous promoter in a chromosome. Preferably, the producer cell according to the embodiment of the present invention contains a vector containing a Cap gene under the control of an exogenous promoter and a vector containing a Rep gene under the control of an exogenous promoter in a chromosome. Being contained in a chromosome means that the whole length or a part of the vector is integrated into the chromosome. Since the vector is integrated into the chromosome, the gene is constitutively maintained in the cell, and the adeno-associated virus can be produced at an optional timing.

Examples of a preferred aspect of the producer cell according to the embodiment of the present invention can include a producer cell containing, in a chromosome, a Cap gene under a control of an exogenous promoter, a Rep gene under a control of an exogenous promoter, an E4 gene under a control of an exogenous promoter, and an E2A gene under a control of an exogenous promoter, in which the producer cell does not contain a VA-RNA gene, the producer cell is a mammalian cell, the Cap gene, the Rep gene, and an E2A gene are present in a vicinity of each other (for example, at the same genetic locus), and the E4 gene is not present in the vicinity of the Cap gene, the Rep gene, and the E2 gene (for example, at the same genetic locus); and a producer cell comprising, in a chromosome, a first vector carrying a Cap gene under a control of an exogenous promoter, a Rep gene under a control of an exogenous promoter, and an E2A gene under a control of an exogenous promoter, and a second vector carrying an E4 gene under the control of an exogenous promoter, in which the producer cell does not contain a VA-RNA gene, and the producer cell is a mammalian cell.

In the producer cell according to the embodiment of the present invention, the number of E4 gene insertions is preferably smaller than the number of insertions of other genes, on the chromosome. The toxicity of E4 causes a decrease in cell survival, and the reason is that the fewer the number of E4 gene insertions is, the easier the cells survive.

In the producer cell according to the embodiment of the present invention, the number of E4 gene insertions on the chromosome is preferably smaller than the number of Cap gene insertions on the chromosome or the number of E2 gene insertions on the chromosome. More preferably, the number of the E4 gene insertions on the chromosome is more preferably 0.8 times or less, still more preferably 0.6 times or less, even still more preferably 0.4 times or less, yet even still more preferably 0.3 times or less, and particularly preferably 0.2 times or less the number of the Cap gene insertions or the number of the E2 gene insertions on the chromosome.

The lower limit of the number of the E4 gene insertions on the chromosome is preferably 0.01 times or more, more preferably 0.02 times or more, and still more preferably 0.03 times or more the number of the Cap gene insertions or the number of the E2 gene insertions on the chromosome.

### <Manufacturing method of producer cell>

The producer cell according to the embodiment of the present invention can be manufactured by transferring a Cap gene under the control of an exogenous promoter and a Rep gene under the control of an exogenous promoter into a mammalian cell. That is, the manufacturing method of a producer cell according to the present invention is a method including transferring a Cap gene under the control of an exogenous promoter and a Rep gene under the control of an exogenous promoter into a mammalian cell.

Preferred aspects of the combination of vectors used in the manufacture of the producer cell according to the embodiment of the present invention include the following aspects.
(1) a vector containing a Rep gene and a Cap gene, a vector containing an E2 (preferably, E2A) gene, and a vector containing a gene for desired treatment or prevention
(2) a vector containing a Rep gene, a Cap gene, and an E2 (preferably, an E2A) gene, and a vector containing a gene for desired treatment or prevention
(3) a vector containing a Rep gene, a Cap gene, an E2 (preferably, an E2A) gene, and a gene for desired treatment or prevention

The vector containing the Cap gene under the control of the exogenous promoter and the vector containing the Rep gene under the control of the exogenous promoter need only to be transferred to be expressed, but in the present invention, the vector is integrated into the chromosome of the cell, thereby the expression is preferably permanent. The permanent expression means that, in a case where a cell is divided, a vector containing the Cap gene under the control of the exogenous promoter and the Rep gene under the control of the exogenous promoter is also replicated, and the divided cell also has the vector.

A vector containing the Cap gene under the control of an exogenous promoter and a vector containing the Rep gene under the control of an exogenous promoter can be incorporated into a chromosome by transfection or transduction. Furthermore, by using the transposon method in combination, the vector can be incorporated into the chromosome with high efficiency.

Transfection refers to transfer of a nucleic acid into a cell across a membrane of eukaryotic cells by chemical means (for example, calcium phosphate mediated precipitation or lipofection), mechanical means (for example, electroporation), or physical means (for example, delivery by bioballistic). Lipofection refers to transfer of a nucleic acid into a cell by the formation of a complex between a vector and a positively charged lipid or the like by electrical interaction, and endocytosis or membrane fusion.

Transduction refers to transfer of a nucleic acid into a cell across a membrane of a eukaryotic cell via a virus-derived vector.

The manufacturing method of the producer cell according to the embodiment of the present invention may include introducing a gene expression regulatory factor, and may further include introducing a reverse tetracycline-controlled transactivator.

The introduction of the reverse tetracycline-controlled transactivator can be performed by transferring a vector containing a gene encoding the reverse tetracycline-controlled transactivator into the cell by a method such as transfection, transduction, or lipofection.

### <Manufacturing method of adeno-associated virus>

In the present invention, the adeno-associated virus can be produced by culturing the producer cell according to the embodiment of the present invention. That is, the manufacturing method of an adeno-associated virus according to the embodiment of the present invention is a method including producing an adeno-associated virus by culturing the producer cell according to the embodiment of the present invention.

Preferably, a step of culturing the producer cell according to the embodiment of the present invention under a condition in which expression of the Cap gene and expression of the Rep gene are not caused to be induced to proliferate the producer cell can be performed, and then a step of culturing the proliferated producer cell under a condition in which expression of the Cap gene and expression of the Rep gene are induced can be performed to produce the adeno-associated virus.

Furthermore, preferably, a step of culturing the producer cell according to the embodiment of the present invention, which contains the E4 gene under the control of an exogenous promoter in a chromosome and does not contain the VA-RNA gene, under a condition in which expression of the Cap gene, expression of the Rep gene, and expression of the E4 gene are not caused to be induced to proliferate the producer cell can be performed, and then a step of culturing the proliferated producer cell under a condition in which expression of the Cap gene, expression of the Rep gene, and expression of the E4 gene are induced can be performed to produce the adeno-associated virus.

The under the condition in which expression of the Cap gene and expression of the Rep gene are not caused to be induced in the producer cell according to the embodiment of the present invention means that the Cap gene and the Rep gene are controlled by an inducible promoter and expression thereof is off, preferably means that the expression thereof is controlled by a drug and the producer cell does not come into contact with the drug for turning on the inducible promoter, and more preferably means that the producer cell does not come into contact with the tetracycline-based drug.

The under condition in which the expression of the Cap gene and the expression of the Rep gene are induced means that the inducible promoter controls the Cap gene and the Rep gene and the expression thereof is on, preferably means that the expression thereof is controlled by a drug and the producer cell comes into contact with the drug for turning on the inducible promoter, more preferably means that the producer cell comes into contact with a tetracycline-based drug, and particularly preferably means that the producer cell comes into contact with doxycycline.

A step of culturing the producer cells according to the present invention under a condition that the expression of the Cap gene and the expression of the Rep gene are not caused to be induced to proliferate the producer cells for 3 days or more can be performed, and then a step of culturing the proliferated producer cells under a condition that the expression of the Cap gene and the expression of the Rep gene are induced to produce the adeno-associated virus can be performed.

Under conditions in which the expression of the Cap gene and the expression of the Rep gene are not caused to be induced, the producer cells are preferably cultured for 3 days or more, more preferably cultured for 5 days or more, and still more preferably cultured for 7 days or more.

In addition, in a case where the producer cell is cultured under a condition in which the expression of the Cap gene and the expression of the Rep gene are induced by bringing the producer cell into contact with doxycycline that turns off the inducible promoter and then removing the doxycycline, the producer cell can produce AAV in a case where the producer cell contains a gene that expresses a tetracycline-controlled transactivator (tTA). The above-described operation has an advantage that the produced AAV is less likely to be contaminated with the antibacterial agent, doxycycline.

The cell culture in the manufacturing method of an adeno-associated virus according to the embodiment of the present invention can be performed under normal conditions for cell culture. The culture medium to be used and the culture conditions can be appropriately selected by those skilled in the art. As the culture medium, the medium includes Expi293 Expression Medium (Thermo Fisher Scientific, A1435101), a Dulbecco's Modified Eagle's Medium (DMEM) containing 10% (vol/vol) fetal bovine serum (FBS), and a serum-free UltraCULTURE (trademark) medium (Lonza) can be used, but the present invention is not particularly limited.

The culture temperature is generally 25°C to 45°C, preferably 30°C to 42°C, more preferably 35°C to 40°C, and 37°C as an example.

The CO₂ concentration is generally 3 to 10% CO₂, preferably 5 to 10% CO₂, and 8% CO₂ as an example.

The cells can be cultured in a culture medium of any volume, for example, the cells can be cultured in a culture medium of 1 mL to 2,000 L, preferably 1 L to 2,000 L, more preferably 50 L to 2,000 L, and particularly preferably 500 L to 2,000 L.

The culture may be carried out while stirring by shaking. The stirring speed in the case of stirring by shaking is generally 50 rpm to 200 rpm, and preferably 80 to 150 rpm.

The stirring culture may be rotary stirring culture using a propeller or the like in a reactor. The stirring speed in the case of stirring by rotating is generally 50 rpm to 200 rpm, and preferably 80 to 150 rpm. In addition, stirring may be performed by wave-type shaking stirring or vertical movement of the stirring blade, but the stirring is not particularly limited.

The culture time in AAV production step is not particularly limited, but is generally 6 hours to 14 days, preferably 12 hours to 7 days, more preferably 24 hours to 144 hours, and still more preferably 24 hours to 96 hours.

The titer of the manufactured adeno-associated virus can be measured by a conventional method known to those skilled in the art. For example, the cell culture solution after the culture is collected, the cells are disrupted by freeze-thaw, and then the supernatant is collected by centrifugation. The gDNA (genomic DNA) and the residual plasmid can be digested by adding MgCl₂ and Benzonase to the collected supernatant to react. It is possible to measure the titer of AAV by performing ddPCR (Droplet Digital PCR) using the sample containing the adeno-associated virus obtained above as a ddPCR sample.

The present invention will be described in more detail with reference to the following examples, but the present invention is not limited to the examples.

### Examples

### [Materials and methods]

### <Cell culture>

Floating HEK293 cells (Thermo Fisher Scientific, R79007) were suspended in 12 mL of Expi293 Expression Medium (Thermo Fisher Scientific, A1435101) in a 1 × 10⁷ cells/mL, and cultured in 125 mL shaking flask for 24 hours. The culture solution was incubated under the conditions of 37°C and 8% CO₂ while being constantly stirred at 120 rpm.

### <Plasmid construction>

AAVpro (registered trademark) Helper Free System (AAV5) (Takara Bio Inc., 6650) was used (SEQ ID NO: 1, SEQ ID NO: 2, and SEQ ID NO: 3).

The entire sequences of the used plasmids are set forth in SEQ ID NOs: 4 to 14 in the sequence list. The sequence was manufactured by a total synthesis method.

**[Table 1]**

| SEQ ID | Sequence: description |
|---|---|
| SEQ ID NO: 1 | pHelper: plasmid encoding E2, E4, and VA-RNA genes |
| SEQ ID NO: 2 | pRCS: plasmid encoding Rep and Cap genes |
| SEQ ID NO: 3 | pAAV-LacZ: plasmid encoding LacZ gene between two ITR sequences |
| SEQ ID NO: 4 | pAAV-GFP: plasmid encoding GFP gene between two ITR sequences |
| SEQ ID NO: 5 | pHelper Vector ΔE4: plasmid in which E4 gene is removed from SEQ ID NO:1 |
| SEQ ID NO: 6 | pHelper Vector ΔVA: plasmid in which VA-RNA gene is removed from SEQ ID NO: 1 |
| SEQ ID NO: 7 | pCMV-CIR: plasmid encoding Cap gene under CMV promoter, insulator sequence, and Rep gene under CMV promoter |
| SEQ ID NO: 8 | pCMV-E2: plasmid encoding E2 gene under CMV promoter |
| SEQ ID NO: 9 | pTET-CIE REP: plasmid encoding Cap gene under TRE-CMV-minimal promoter, IRES sequence, E2 gene, and Rep gene under TRE-CMV-minimal promoter |
| SEQ ID NO: 10 | prtTA: plasmid encoding rtTA gene under CMV promoter |
| SEQ ID NO: 11 | pLenti AAV GFP-Puro: Lentivirus packaging plasmid encoding GFP and puromycin resistant gene between two ITR sequences |
| SEQ ID NO: 12 | pLenti_CMV-E2-Hyg: Lentivirus packaging plasmid encoding E2 gene and hvgromvcin resistant gene under CMV promoter |
| SEQ ID NO: 13 | pTET-CIE-REP-rtTA-REP: plasmid encoding Cap gene under TRE-CMV-minimal promoter, IRES sequence, E2 gene, insulator sequence, Rep gene under TRE-CMV-minimal promoter, rtTA gene under CMV promoter, and RFP gene under CMV promoter |
| SEQ ID NO: 14 | pTET-CIE-REP-rtTA-NEO: plasmid encoding Cap gene under TRE-CMV-minimal promoter, IRES sequence, E2 gene, insulator sequence, Rep gene under TRE-CMV-minimal promoter, rtTA gene under CMV promoter, and neomycin resistant gene under PGK promoter |
| SEQ ID NO: 15 | ddPCR-PrimerF: primer for AAV genome detection |
| SEQ ID NO: 16 | ddPCR-PrimerR: primer for AAV genome detection |
| SEQ ID NO: 17 | Probe: probe for AAV genome detection |
| SEQ ID NO: 18 | (Plasmid of gene Design 1 in Fig. 10) |
| | pTET-SRIE2-CIR-rtTA-Puro-GOI: plasmid encoding Small Rep gene under TRE-CMV-minimal promoter, IRES sequence, E2 gene, Cap gene under TRE-CMV-minimal promoter, IRES sequence, Rep gene, rtTA gene under CMV promoter, IRES sequence, puromycin resistant gene, and GFP gene between two ITR sequences, between piggyBac transposase recognition sequences |
| SEQ ID NO: 19 | (Plasmid of gene Design 2 in Fig. 10) |
| | pTET-CIE2-SRIR-rtTA-Puro-GOI: plasmid encoding Cap gene under TRE-CMV-minimal promoter, IRES sequence, E2 gene, Small Rep gene under TRE-CMV-minimal promoter, IRES sequence, Rep gene, rtTA gene under CMV promoter, IRES sequence, puromycin resistant gene, and GFP gene between two ITR sequences, between piggyBac transposase recognition sequences |
| SEQ ID NO: 20 | (Plasmid of gene Design 3 in Fig. 10) first vector |
| | pTET-C-SRIR-rtTA-Puro-GOI: plasmid encoding Cap gene under TRE-CMV-minimal promoter, Small Rep gene under TRE-CMV-minimal promoter, IRES sequence, Rep gene, rtTA gene under CMV promoter, IRES sequence, puromycin resistant gene, and GFP gene between two ITR sequences, between piggyBac transposase recognition sequences |
| SEQ ID NO: 21 | (Plasmid of gene Design 3 in Fig. 10) second vector |
| | pTET-E2: plasmid encoding E2 gene under TRE-CMV-minimal promoter, rtTA gene under EF-1α promoter, and neomycin resistant gene under PGK promoter |
| SEQ ID NO: 22 | (Plasmid of gene Design 4 in Fig. 10) first vector |
| | pTET-CIE2-SRIR-rtTA-Puro-GOI: plasmid encoding Cap gene under TRE-CMV-minimal promoter, IRES sequence, E2 gene, Small Rep gene under TRE-CMV-minimal promoter, IRES sequence, Rep gene, rtTA gene under CMV promoter, IRES sequence, puromycin resistant gene, and GFP gene between two ITR sequences, between piggyBac transposase recognition sequences |
| SEQ ID NO: 23 | (Plasmid of gene Design 4 in Fig. 10) second vector |
| | pTET-REP-IE4: plasmid encoding RFP gene under TRE-CMV-minimal promoter, IRES sequence, E4 gene, and neomycin resistant gene under PGK promoter |
| SEQ ID NO: 24 | (Plasmid of gene Design 5 in Fig. 10) |
| | pTET CIE2IE4-SRIR-rtT.A-Puro-GOI: plasmid encoding Cap gene under TRE-CMV-minimal promoter, IRES sequence, E2 gene, IRES sequence, E4 gene, Small Rep gene under TRE-CMV-minimal promoter, IRES sequence, Rep gene, rtTA gene under CMV promoter, IRES sequence, puromycin resistant gene, and GFP gene between two ITR sequences, between piggyBac transposase recognition sequences |
| SEQ ID NO: 25 | (Plasmid of gene Design 6 in Fig. 10) first vector |
| | pTET-C-SRIR-rtTA-Puro-GOI: plasmid encoding Cap gene under TRE-CMV-minimal promoter, Small Rep gene under TRE-CMV-minimal promoter, IRES sequence, Rep gene, rtTA gene under CMV promoter, IRES sequence, puromycin resistant gene, and GFP gene between two ITR sequences, between piggyBac transposase recognition sequences |
| SEQ ID NO: 26 | (Plasmid of gene Design 6 in Fig. 10) second vector |
| | pTET-E2-IE4: plasmid encoding E2 gene under TRE-CMV-minimal promoter, IRES sequence, E4 gene, rtTA gene under EF-1α promoter, and neomycin resistant gene under PGK promoter |
| SEQ ID NO: 27 | Plasmid encoding piggyBac transposase gene under CMV promoter |
| SEQ ID NO: 28 | ddPCR-RPP30 F: primer 1 for RPP30 gene detection |
| SEQ ID NO: 29 | ddPCR-RPP30 R: primer 2 for RPP30 gene detection |
| SEQ ID NO: 30 | ddPCR-RPP30 P: probe for RPP30 gene detection |
| SEQ ID NO: 31 | ddPCR-Rep F: primer 1 for Rep gene detection |
| SEQ ID NO: 32 | ddPCR-Rep R: primer 2 for Rep gene detection |
| SEQ ID NO: 33 | ddPCR-Rep P: probe for Rep gene detection |
| SEQ ID NO: 34 | ddPCR-Cap F: primer 1 for Cap gene detection |
| SEQ ID NO: 35 | ddPCR-Cap R: primer 2 for Cap gene detection |
| SEQ ID NO: 36 | ddPCR-Cap P: probe for Cap gene detection |
| SEQ ID NO: 37 | ddPCR-E2 F: primer 1 for E2 gene detection |
| SEQ ID NO: 38 | ddPCR-E2 R: primer 2 for E2 gene detection |
| SEQ ID NO: 39 | ddPCR-E2 P: probe for E2 gene detection |
| SEQ ID NO: 40 | ddPCR-E4 F: primer 1 for E4 gene detection |
| SEQ ID NO: 41 | ddPCR-E4 R: primer 2 for E4 gene detection |
| SEQ ID NO: 42 | ddPCR-E4 P: probe for E4 gene detection |

### <Gene transfer method>

36 µL of Polyethylenimine (PEI) (PolyPlus-transfection SAS, 115-0015) and an equal amount of the plasmids of the combination described below were mixed, and a total amount of 18 µg of the mixture was suspended in 1.8 mL of Expi293 Expression Medium and allowed to stand for 15 minutes. Then, the above-mentioned reagent was added to the cultured cells (culture solution volume was 12 mL) seeded at a cell concentration of 1 × 10⁷ cells/mL on the previous day, and the cells were incubated under the conditions of 37°C and 8% CO₂.

### <Western blot>

Cells were collected by centrifugation and lysed with a RIPA Buffer. Sample Buffer (nacalai tesque, 09499-14) was added to the cell solution and the mixture was heated at 100°C for 5 minutes to prepare a sample. The protein in the sample was size-separated using a sodium dodecyl sulfate (SDS) gel electrophoresis device (ATTO CORPORATION, 2321670), and then transferred to a membrane using a semi-dry blotting device (ATTO CORPORATION, 2322490). The membrane after transfer was blocked with Blocking One (nacalai tesque, 03953-95) for 30 minutes, then reacted with an anti-REP antibody (OriGene Technologies) diluted 70-fold with a blocking solution, an anti-VP antibody (PROGEN Biotechnik GmbH) that reacts with a capsid structural protein, and an anti-β-Actin antibody (Abcam) at room temperature for 3 hours. Next, the reactant was reacted with anti-mouse IgG-HRP (Cytiva), as a secondary antibody, diluted 10,000-fold with a blocking solution at room temperature for 2 hours, and then the REP protein and the capsid structural proteins (VP1, VP2, VP3) were detected with a SuperSignal West Femto Maximum Sensitivity Substrate (Thermo Fisher Scientific, 34095). WSE-6100 LuminoGraphI (ATTO CORPORATION, 2006100) was used for imaging. Quantitative evaluation was performed using ImageJ for the analysis of the acquired image.

### <Measurement of AAV titer>

The HEK cell culture solution, which had been cultured for 72 hours after the gene transfer under the conditions of 37°C and 8% CO₂, was collected, and the cells were disrupted by freezing and thawing at -80°C. Then, the supernatant was collected by centrifugation at 13,800 ×g, 2 mmol/L (final concentration) MgCl₂, and 25 U/mL Benzonase was added thereto, and a reaction was carried out at 37°C for 2 hours to digest gDNA (genomic DNA) and residual plasmid. The sample after the reaction was incubated at 95°C for 15 minutes, 70°C for 3 minutes, 40°C for 3 minutes, and 20°C for 3 minutes in this order, to inactivate Benzonase, and the reactant was used as a ddPCR (Droplet Digital PCR) sample. The ddPCR sample was diluted 50-fold with a TE buffer (pH 8.0, containing 0.05% Pluronic F-68 and 10 µg/mL bovine thymus DNA). 10 µL of ddPCRtm Supermix for Probes (no dUTP) (Bio-Rad Laboratories, Inc.), 2 µL of diluted ddPCR sample, primers (SEQ ID NO 15, SEQ ID NO 16, final concentration of 900 nmol/L) and probe (SEQ ID NO 17, final concentration of 250 nmol/L), nuclease free water (Thermo Fisher Scientific, 10977015) was mixed in a tube on ice, the total liquid volume was adjusted to 22 µL, and ddPCR was performed.

In ddPCR, the droplets were formed from the preparation liquid by a droplet forming apparatus, and subjected to denaturation, annealing, and extension at 95°C for 10 minutes (preheating), at 94°C for 30 seconds (denaturation), and at 55°C for 60 seconds (annealing and extension) for 40 cycles and incubated at 98°C for 10 minutes, in this order. Measurement was performed using a droplet reader, and the AAV genomic titer (vg/mL) was calculated.

### <Establishment of cell strain>

36 µL of Polyethylenimine (PEI) (PolyPlus-transfection SAS, 115-0015) and an equal amount of the plasmids of the combination described below were mixed, and a total amount of 18 µg of the mixture was suspended in 1.8 mL of Expi293 Expression Medium and allowed to stand for 15 minutes. Then, the above-mentioned reagent was added to the cultured cells (culture solution volume was 12 mL) seeded at a cell concentration of 1 × 10⁷ cells/mL on the previous day, and the cells were incubated under the conditions of 37°C and 8% CO₂. The gene-transferred cells were maintained and cultured for 3 weeks, and RFP or GFP fluorescence-positive cells were isolated as single cells using drug selection with a resistant gene or flow cytometry. The isolated cells were suspended in DMEM containing 10% fetal calf serum and cultured on a 6-well plate under the conditions of 37°C and 8% CO₂ for 1 month to establish a cell strain.

After gene transfer, doxycycline (DOX) was added to the sample of DOX (+) in a final concentration of 0.5 µg/mL. The AAV genomic titer extracted from HEK293 cells cultured for 72 hours after the addition of doxycycline was evaluated.

### Experiment 1:

### <Method>

Each plasmid having the sequence described below was gene transferred into cells, the cells were collected, and the expression was evaluated by Western blotting.
Ctrl: SEQ ID NO: 1, SEQ ID NO: 2, and SEQ ID NO: 3
ΔHelper: SEQ ID NO: 2 and SEQ ID NO: 3
ΔE4: SEQ ID NO: 2, SEQ ID NO: 3, and SEQ ID NO: 5
ΔVA-RNA: SEQ ID NO: 2, SEQ ID NO: 3, and SEQ ID NO: 6

### <Result>

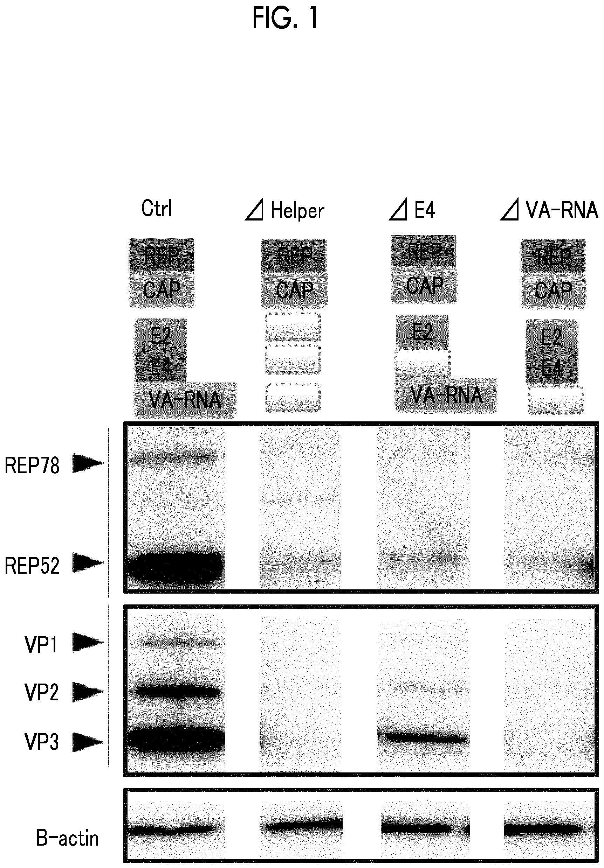

The results are shown in Fig. 1.

In a case where the helper genes (three types) were absent (Δhelper), REP and CAP were not expressed. In addition, also in a case where E4 or VA-RNA was removed alone, the expression levels of REP and CAP were significantly reduced.

In commercially available kits (Takara or the like), it is common to use three types of plasmids, but various genes contained in these plasmids interact with each other. Therefore, in a case where the E4 gene and the VA-RNA gene are not used, REP and CAP cannot be caused to be expressed, and AAV cannot be produced.

### Experiment 2:

### <Method>

Each plasmid having the sequence described below was gene transferred into cells, the cells were collected after 72 hours, and then the AAV production ability was evaluated by ddPCR.
Ctrl: SEQ ID NO: 1, SEQ ID NO: 4, and SEQ ID NO: 7
ΔE4ΔVA-RNA: SEQ ID NO: 4, SEQ ID NO: 7, and SEQ ID NO: 8

### <Result>

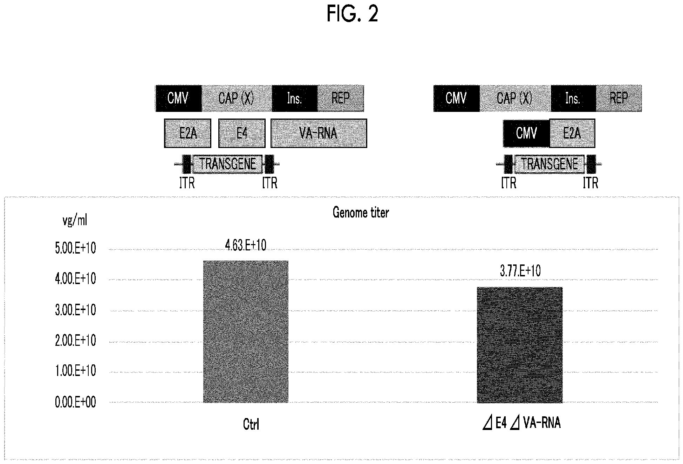

The results are shown in Fig. 2.

In a case where the exogenous promoter was bonded to the Rep gene and the Cap gene, about 80% of AAV could be produced even in a case where E4 and VA were absent.

### Experiment 3:

### <Method>

The SEQ ID NO: 9 was prepared and transferred into cells at the same time as the SEQ ID NO: 10 was transferred. Doxycycline (DOX) was added 24 hours after the gene transfer, and the expression of the REP protein and the expression of the CAP protein were evaluated by Western blotting 72 hours after the addition.
rtTA(-), DOX(-): a state in which the TET adjustment factor and the inducer were absent (evaluation of gene expression leak in the case where the switch was OFF)
rtTA(+), DOX(-): a state in which the TET adjustment factor was present and the inducer was absent (evaluation of gene expression leak in a case where the switch was OFF)
rtTA(+), DOX(+): a state in which the TET adjustment factor and the inducer were present (evaluation of gene expression in a case where the switch was ON)

The rtTA was a protein that cause a gene under the control of a TRE-CMV-minimal promoter to be expressed, and was introduced as a plasmid. As a promoter of rtTA, a CMVminimal promoter was used.

### <Result>

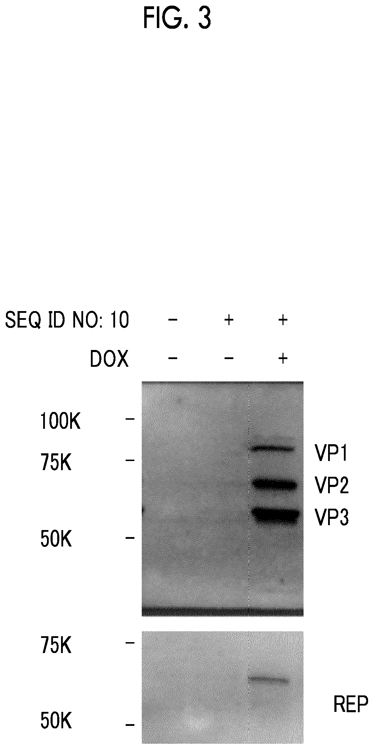

The results are shown in Fig. 3.

In the presence of the SEQ ID NO: 10, the SEQ ID NO: 9 could control the expression of the REP protein and the expression of the CAP protein in a case of adding doxycycline.

### Experiment 4: manufacture of E2 gene and GFP-inserted cells

### <Method>

The SEQ ID NO: 11 or the SEQ ID NO: 12 was transferred into HEK293T cells (Takara) together with Lentiviral High Titer Packaging Mix (Takara) to produce a lentivirus. The culture supernatant was added to 1/100 volume of HEK293 cells together with 2 to 12 µg/ml of polybrene to establish the infected cells. The drug was added thereto, and the genome was extracted from the culture which had been subcultured for one month or more, and insertion of the E2 gene and the GFP gene into the chromosome was confirmed by PCR. Whether or not the protein was expressed was detected by Western Blot (E2 antibody). In addition, it was confirmed that GFP exhibited green fluorescence in a fluorescence microscope.

### <Result>

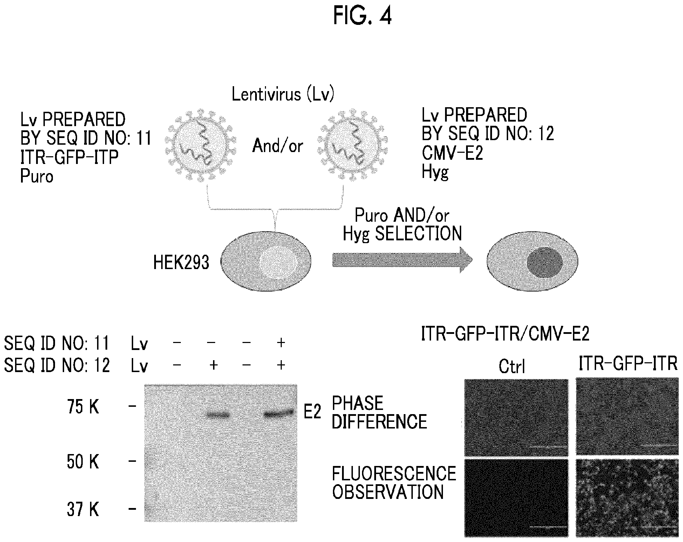

The results are shown in Fig. 4. Fig. 4 shows the results of a Western blot using an anti-E2 antibody (left figure in the lower part of Fig. 4) and a GFP observation (fluorescence microscope) (right figure in the lower part of Fig. 4) for the stable expression strain (proliferated cells).

A stable strain into which GFP flanked by ITR as the target gene was inserted was established, and a stable strain into which E2 driven under a CMV promoter was inserted was established.

### Experiment 5: evaluation of AAV function

### <Method>

A Rep gene and a Cap gene (SEQ ID NO: 7) were inserted with a plasmid into the cell strain into which the GFP flanked by CMV-E2 and ITR, which were described above, were inserted. After 72 hours from the introduction, the culture supernatant was collected, and the AAV was detected by ddPCR.

### <Result>

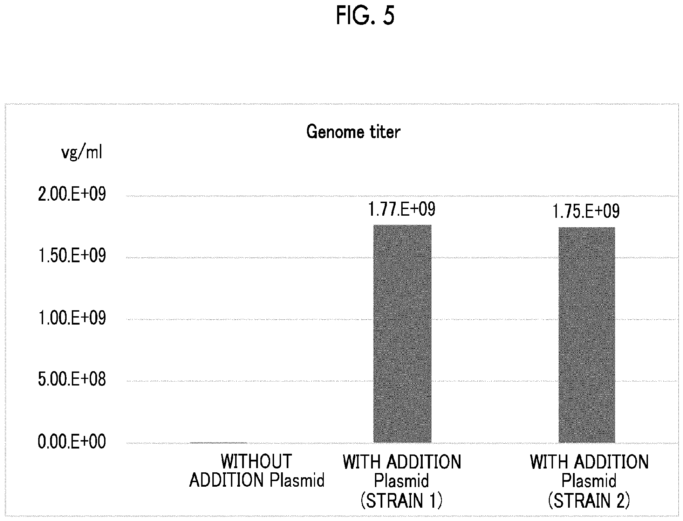

The results are shown in Fig. 5.

In a case where SEQ ID NO: 7 was not added, AAV was hardly detected, but in the cell strains (two types of strains established on a separate day) into which the SEQ ID NO: 7 was transferred, AAV could be produced.

### Experiment 6: manufacture of producer cell

### <Method>

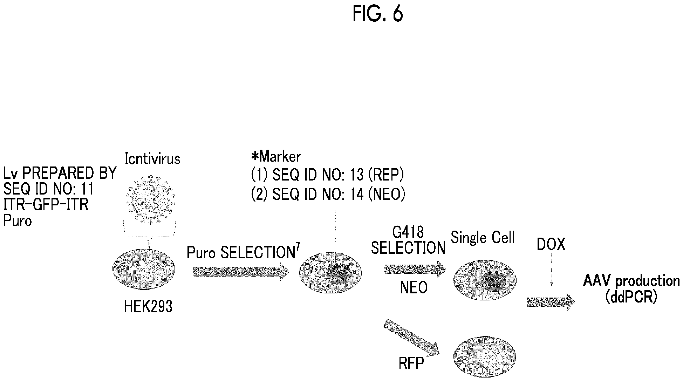

The lentivirus was created using SEQ ID NO: 11, and the cell obtained by gene transferring SEQ ID NO: 13 (selection marker: RFP, red fluorescent protein) into HEK293 infection transferred by the method described in <Method> of "Experiment 4: manufacture of E2 gene and GFP-inserted cells" and the cell obtained by gene transferring SEQ ID NO: 14 (selection marker: NEO, neomycin resistant gene) into the HEK293 were prepared, and single strains in which each of the genes had been transferred into the chromosome were selected. The transferred cell of SEQ ID NO: 13 was selected using red fluorescence as an indicator. In addition, the transferred cell of SEQ ID NO: 14 was selected (Fig. 6) by adding G418 thereto at a concentration of 200 µg/ml. The genome was taken out from the cells cultured for 1 month or more after the gene transfer, and the transfer into the genome was confirmed by a PCR method to establish a stable strain.

The selected cell strains (30 strains of the transferred strain of SEQ ID NO: 14 and 60 strains of the transferred strain of SEQ ID NO: 13) were seeded such that the concentration was 300,000/well, and doxycycline was added to induce gene expression. The AAV was quantified by ddPCR after 72 hours.

### <Result>

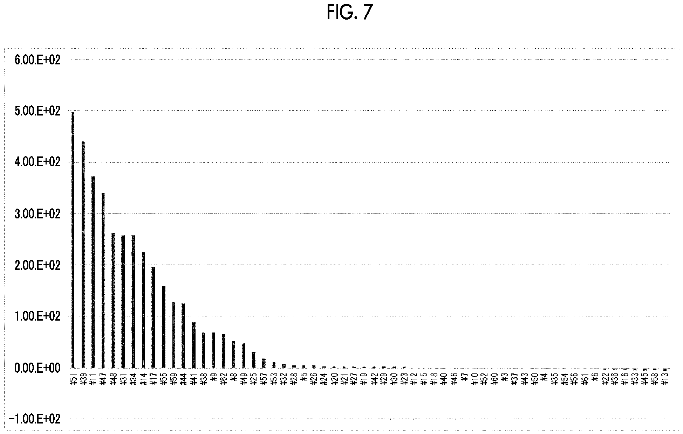

Fig. 7 shows the quantification results of AAV for a strain into which SEQ ID NO: 14 (selection marker: NEO, neomycin resistant gene) was transferred. The vertical axis represents AAV Titer vg/cell, and the horizontal axis represents a clone number.

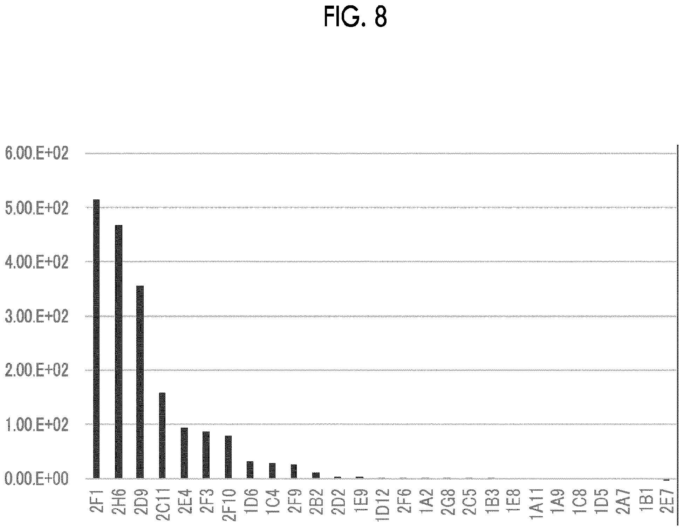

Fig. 8 shows the quantification results of AAV for a strain into which SEQ ID NO: 13 (selection marker: RFP, red fluorescent protein) was transferred. The vertical axis represents AAV Titer vg/cell, and the horizontal axis represents a clone number.

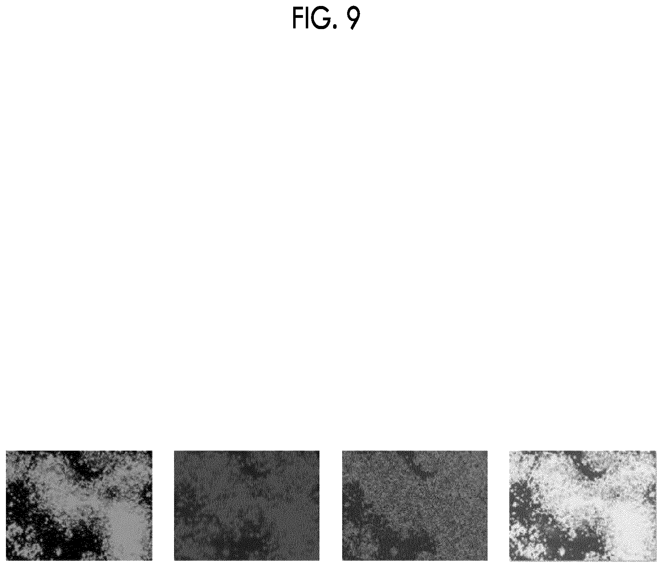

The representative microscopic views of the stable strain were shown in Fig. 9.

### Experiment 7: manufacture of producer cell (2)

### <Method>

A cell (Design 1 transferred cell) obtained by transferring SEQ ID NO: 18 (selection marker: GFP) into HEK293, a cell (Design 2 transferred cell) obtained by transferring SEQ ID NO: 19 (selection marker: GFP) into HEK293, a cell (Design 3 transfer cell) obtained by transferring SEQ ID NO: 20 and SEQ ID NO: 21 (selection marker and drug: GFP and neomycin resistant gene (NEO)) into HEK293, a cell (Design 4 transfer cell) obtained by transferring SEQ ID NO: 22 and SEQ ID NO: 23 (selection marker and drug: GFP and NEO) into HEK293, a cell (Design 5 transfer cell) obtained by transferring SEQ ID NO: 24 (selection marker: GFP) into HEK293, and a cell (Design 6 transfer cell) obtained by transferring SEQ ID NO: 25 and SEQ ID NO: 26 (selection marker and drug: GFP and NEO) into HEK293 were prepared, and single strains in which each of the genes had been transferred into the chromosome were selected.

At the time of preparation of Design 1 to Design 6 transferred cells, in order to improve the recombination efficiency into the chromosomes of SEQ ID Nos: 18, 19, 20, 22, 24, and 25, the plasmid vector of SEQ ID NO: 27 was further co-introduced and the transposase recombinase enzyme was transiently expressed.

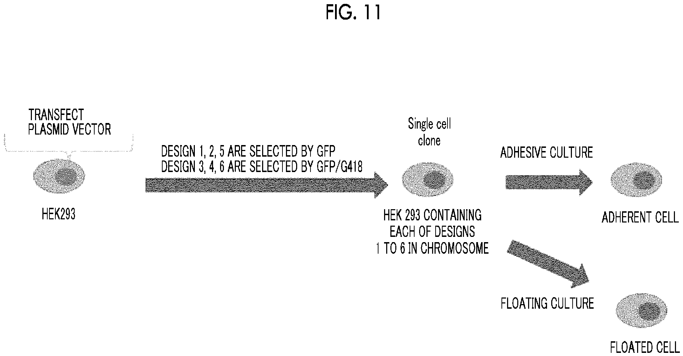

Design 1 transferred cell, Design 2 transferred cell, and Design 5 transferred cell were selected using green fluorescence as an indicator. In addition, Design 3 transferred cell, Design 4 transferred cell, and Design 6 transferred cell were selected by adding the G418 thereto at a concentration of 200 µg/ml, and were further selected using green fluorescence as an indicator (Fig. 11).

The genome was taken out from the cells cultured for 1 month or more after the gene transfer, and the transfer into the genome was confirmed by a PCR method to establish a stable strain.

The selected cell strain was seeded on a 24-well plate such that the concentration was 3.0 × 10⁵ cells/well, and doxycycline was added thereto to induce gene expression. The AAV was quantified by ddPCR after 72 hours.

For the selected cell strain, 1.0 × 10⁶ cells of the cells were collected, and the genomic DNA was extracted using a DNeasy Blood & Tissue Kit (QIAGEN). This was used as a ddPCR sample. The ddPCR sample was diluted to 15 ng/µl with nuclease-free water (Thermo Fisher Scientific, 10977015). 10 µL of ddPCRtm Supermix for Probes (no dUTP) (Bio-Rad Laboratories, Inc.), 2 µL of diluted ddPCR sample, primers (final concentration of 900 nmol/L) and probe (final concentration of 250 nmol/L) in combination described in Table 2, nuclease free water (Thermo Fisher Scientific, 10977015) were mixed in a tube on ice, the total liquid volume was adjusted to 22 µL, and ddPCR was performed. The number of gene insertions on the genome per cell was calculated from the ddPCR measurement value of the genomic DNA sample by the following Expression 1. <Expression 1> Number of gene insertions on genome per cell (copies/cell) = (quantification number of FAM/quantification number of HEX) × 3

**[Table 2]**

| | |
|---|---|
| Detection set for number of Rep genes | SEQ ID NO: 28, SEQ ID NO: 29, SEQ ID NO: 30 |
| | SEQ ID NO: 31, SEQ ID NO: 32, SEQ ID NO: 33 |
| Detection set for number of Cap genes | SEQ ID NO: 28, SEQ ID NO: 29, SEQ ID NO: 30 |
| | SEQ ID NO: 34, SEQ ID NO: 35, SEQ ID NO: 36 |
| Detection set for number of E2 genes | SEQ ID NO: 28, SEQ ID NO: 29, SEQ ID NO: 30 |
| | SEQ ID NO: 37, SEQ ID NO: 38, SEQ ID NO: 39 |
| Detection set for number of E4 genes | SEQ ID NO: 28, SEQ ID NO: 29, SEQ ID NO: 30 |
| | SEQ ID NO: 40, SEQ ID NO: 41, SEQ ID NO: 42 |

HEK fluorescent dye-labeled DNA probes were used for SEQ ID NO: 30, and FAM fluorescent dye-labeled DNA probes were used for SEQ ID NO: 33, SEQ ID NO: 36, SEQ ID NO: 39, and SEQ ID NO: 42.

### <Result>

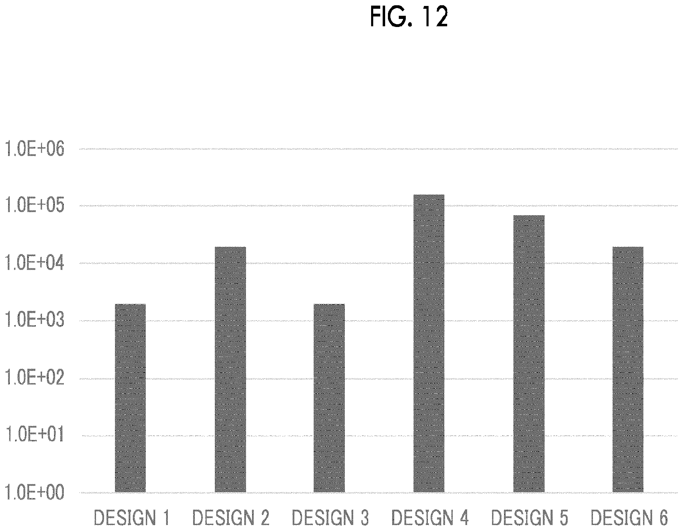

Fig. 12 shows the quantification results of AAV for the highest titer strain of Design 1 to Design 6 transferred cells. The vertical axis represents AAV Titer vg/cell, and the horizontal axis represents the name of the cell strain.

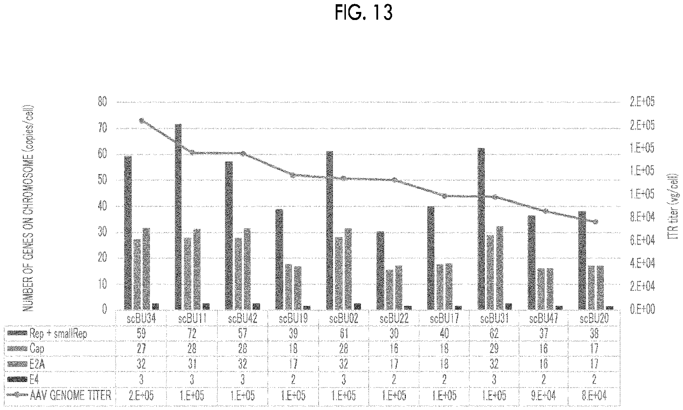

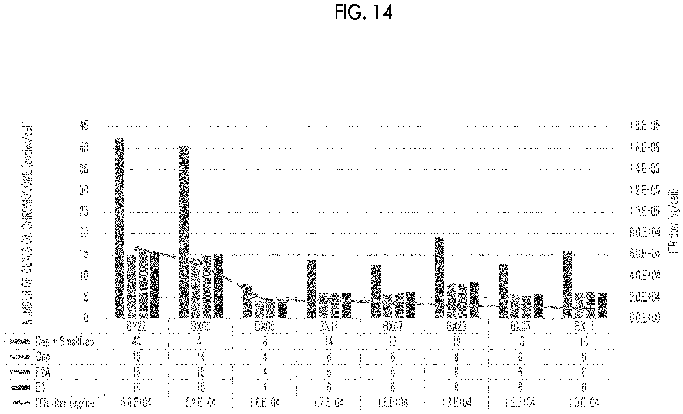

The number of gene insertions on the genome measured by the PCR method for Designed 4 transferred cell strains (10 strains) and Designed 5 transferred cell strains (10 strains) was shown in Fig. 13 and Fig. 14. The first vertical axis represents the number of gene insertions per cell, the second vertical axis represents AAV Titer vg/cell, and the horizontal axis represents the clone name.

### Experiment 8: floating of producer cell

### <Method>

The highest titer strain of Design 4 transferred cell and the highest titer strain of Design 5 transferred cell, which had been established in Experiment 7, were suspended in 12 mL of Expi293 Expression Medium (Thermo Fisher Scientific, A1435101) such that the cell concentration was 0.1 to 2 × 10⁶ cells/mL, and the suspension was cultured and floated in a 125 mL shaking flask. The culture solution was incubated under the conditions of 37°C and 8% CO₂ while being constantly stirred at 120 rpm. The producer cells were continuously cultured for 1 month after the floating.

The cell strain that had been subjected to the suspension culture was seeded in Expi293 Expression Medium such that the concentration was 4.0 × 10⁵ cells/mL, and doxycycline was added to induce gene expression. The culture solution was incubated under the conditions of 37°C and 8% CO₂ while being constantly stirred at 120 rpm. The AAV was quantified by ddPCR after 72 hours.

### <Result>

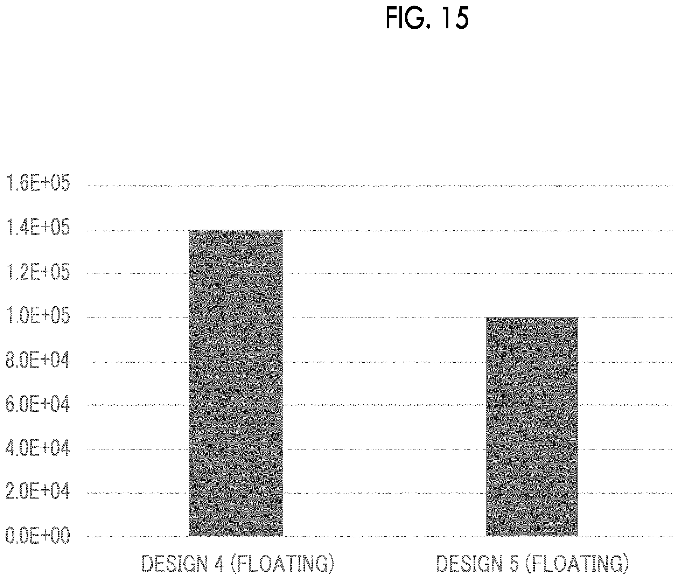

Fig. 15 shows the quantification results of AAV for the highest titer strain of the floated Design 4 transferred cell and the highest titer strain of the floated Design 5 transferred cell. The vertical axis represents AAV Titer vg/cell, and the horizontal axis represents the name of the cell strain.

### [Sequence list]

International application 21F01631W1JP23008736_5.xml based on International Patent Cooperation Treaty

## Claims

1. A producer cell comprising, in a chromosome:
a Cap gene under a control of an exogenous promoter; and
a Rep gene under a control of an exogenous promoter,
wherein the producer cell does not contain at least one of a VA-RNA gene or an E4 gene, and
the producer cell is a mammalian cell.

2. The producer cell according to claim 1,
wherein the exogenous promoter of the Rep gene and the exogenous promoter of the Cap gene are the same promoter.

3. The producer cell according to claim 1,
wherein the Rep gene is present on a downstream side of the Cap gene in a transcription direction.

4. The producer cell according to claim 1,
wherein the producer cell contains the E4 gene under a control of an exogenous promoter in the chromosome, and
the producer cell does not contain the VA-RNA gene.

5. The producer cell according to claim 4,
wherein the E4 gene is present on a downstream side of an IRES.

6. The producer cell according to claim 4,
wherein the exogenous promoter of the E4 gene is an inducible promoter capable of switching on and off

7. The producer cell according to claim 6,
wherein the exogenous promoter of the E4 gene is a promoter capable of inducing expression by a drug.

8. The producer cell according to claim 7,
wherein the exogenous promoter of the E4 gene is present on a downstream side of a tetracycline response element.

9. The producer cell according to claim 1,
wherein the producer cell does not contain both the VA-RNA gene and the E4 gene.

10. The producer cell according to any one of claims 1 to 9, further comprising:
an E2A gene.

11. The producer cell according to claim 10,
wherein the E2A gene is present on a downstream side of an IRES.

12. The producer cell according to claim 10,
wherein the E2A gene is under a control of an exogenous promoter.

13. The producer cell according to claim 12,
wherein the E2A gene is under a control of a cytomegalovirus-derived promoter.

14. The producer cell according to claim 10,
wherein an exogenous promoter of the E2A gene is an inducible promoter capable of switching on and off.

15. The producer cell according to claim 14,
wherein the exogenous promoter of the E2A gene is a promoter capable of inducing expression by a drug.

16. The producer cell according to claim 15,
wherein the exogenous promoter of the E2A gene is present on a downstream side of a tetracycline response element.

17. A producer cell comprising, in a chromosome:
a Cap gene under a control of an exogenous promoter;
a Rep gene under a control of an exogenous promoter; and
an E4 gene under a control of an exogenous promoter,
wherein the producer cell does not contain a VA-RNA gene, and
the producer cell is a mammalian cell.

18. The producer cell according to any one of claims 1 to 17, further comprising:
a target gene.

19. The producer cell according to any one of claims 1 to 18,
wherein one or more of the exogenous promoter of the Cap gene and the exogenous promoter of the Rep gene are an inducible promoter capable of switching on and off.

20. The producer cell according to claim 19,
wherein the one or more of the exogenous promoter of the Cap gene and the exogenous promoter of the Rep gene are a promoter capable of inducing expression by a drug.

21. The producer cell according to claim 20,
wherein the one or more of the exogenous promoter of the Cap gene and the exogenous promoter of the Rep gene is present on a downstream side of a tetracycline response element.

22. The producer cell according to any one of claims 1 to 21, further comprising:
a Small Rep gene under a control of an exogenous promoter.

23. The producer cell according to claim 22,
wherein a Rep gene is present on a downstream side of the Small Rep gene in a transcription direction.

24. The producer cell according to claim 22,
wherein the exogenous promoter of the Small Rep gene is an inducible promoter capable of switching on and off.

25. The producer cell according to claim 24,
wherein the exogenous promoter of the Small Rep gene is a promoter capable of inducing expression by a drug.

26. The producer cell according to claim 25,
wherein the exogenous promoter of the Small Rep gene is present on a downstream side of a tetracycline response element.

27. The producer cell according to any one of claims 1 to 26, further comprising:
a gene that expresses a reverse tetracycline-controlled transactivator.

28. The producer cell according to claim 27,
wherein the gene that expresses the reverse tetracycline-controlled transactivator is present on a downstream side of the Rep gene.

29. The producer cell according to claim 1,
wherein the producer cell contains an E4 gene under a control of an exogenous promoter, an E2A gene under a control of an exogenous promoter, and a Small Rep gene under a control of an exogenous promoter, and
the exogenous promoter of the Cap gene and an exogenous promoter of an E2A gene are the same promoter, and the exogenous promoter of the Rep gene and the exogenous promoter of the Small Rep gene are the same promoter.

30. A producer cell comprising, in a chromosome:
a Cap gene under a control of an exogenous promoter;
a Rep gene under a control of an exogenous promoter;
an E4 gene under a control of an exogenous promoter; and
an E2A gene under a control of an exogenous promoter,
wherein the producer cell does not contain a VA-RNA gene,
the producer cell is a mammalian cell,
the Cap gene, the Rep gene, and an E2A gene are present in a vicinity of each other, and
the E4 gene is not present in the vicinity of the Cap gene, the Rep gene, and the E2A gene.

31. A producer cell comprising, in a chromosome:
a first vector carrying a Cap gene under a control of an exogenous promoter, a Rep gene under a control of an exogenous promoter, and an E2A gene under a control of an exogenous promoter; and
a second vector carrying an E4 gene under a control of an exogenous promoter,
wherein the producer cell does not contain a VA-RNA gene, and
the producer cell is a mammalian cell.

32. The producer cell according to claim 31,
wherein the number of E4 gene insertions is smaller than the number of insertions of other genes, on the chromosome.

33. The producer cell according to claim 31 or 32,
wherein the number of E4 gene insertions on the chromosome is smaller than the number of Cap gene insertions on the chromosome or the number of E2 gene insertions on the chromosome.

34. The producer cell according to claim 31 or 32,
wherein the number of E4 gene insertions on the chromosome is 0.4 times or less the number of Cap gene insertions on the chromosome.

35. The producer cell according to claim 31 or 32,
wherein the number of E4 gene insertions on the chromosome is 0.4 times or less the number of E2 gene insertions on the chromosome.

36. A manufacturing method of a producer cell, which is a manufacturing method of the producer cell according to any one of claims 1 to 35, the manufacturing method comprising:
transferring the Cap gene under the control of the exogenous promoter and the Rep gene under the control of the exogenous promoter into a mammalian cell.

37. The manufacturing method of a producer cell according to claim 36, further comprising:
introducing a reverse tetracycline-controlled transactivator.

38. A manufacturing method of an adeno-associated virus, comprising:
culturing the producer cell according to any one of claims 1 to 35, to produce an adeno-associated virus.

39. A manufacturing method of an adeno-associated virus, comprising:
a step of culturing the producer cell according to any one of claims 1 to 35 under a condition in which expression of the Cap gene and expression of the Rep gene are not caused to be induced, to proliferate the producer cell; and
a step of culturing the proliferated producer cell under a condition in which the expression of the Cap gene and the expression of the Rep gene are induced, to produce an adeno-associated virus.

40. A manufacturing method of an adeno-associated virus, comprising:
a step of culturing the producer cell according to any one of claims 4 to 8 under a condition in which expression of the Cap gene, expression of the Rep gene, and expression of the E4 gene are not caused to be induced, to proliferate the producer cell; and
a step of culturing the proliferated producer cell under a condition in which the expression of the Cap gene, the expression of the Rep gene, and the expression of the E4 gene are induced, to produce an adeno-associated virus.
